# EUROPEAN PATENT APPLICATION

(11) **EP 4 209 575 A1**
(43) Date of publication of application: **12.07.2023**
(21) Application number: 22151042.3
(22) Date of filing: 11.01.2022
(51) Int. Cl.: C12M 1/00, C12M 1/12, C12M 1/26, C12M 1/34, C12M 1/36

(54) **METHOD FOR OPERATING A CLARIFICATION SETUP**

(71) Applicant: Sartorius Stedim North America Inc., Bohemia, NY 11716 (US)
(72) Inventor: Soeldner, Robert, 37083 Göttingen (DE); Austerjost, Jonas, 33397 Rietberg (DE)
(74) Representative: Gottschald Patentanwälte Partnerschaft mbB

(57) **Abstract**

The invention is directed to a method for operating a clarification setup (1) of a bioprocess installation (2), which clarification setup comprises a fluidized bed centrifuge (3) and a pumping arrangement (4), wherein the fluidized bed centrifuge (3) comprises at least one centrifuge chamber (5) turned around a geometrical centrifuge axis (6), wherein the bioprocess installation (2) comprises an electronic process control (7) for controlling the fluidized bed centrifuge (3) and pumping arrangement (4), wherein the fluidized bed centrifuge (3) is being operated in a forward operation for a particle loading cycle (8) and/or a particle washing cycle (9) and in a backward operation for a particle discharging cycle (10), wherein in case where a particle loading cycle (8) is provided, cell broth loaded into the centrifuge chamber (5) proceeds to form a growing particle accumulation in the centrifuge chamber (5), wherein the clarification setup (1) comprises a monitoring sensor arrangement (11) with at least one optical sensor (12) for producing monitoring sensor data, which are being transmitted to the electronic process control (7). It is proposed that in a monitoring routine (13), image-related data (14) representing an optical image (15) of the centrifuge chamber content (16) are being produced by the monitoring sensor arrangement (11) and a particle filling level (17) is being calculated by the electronic process control (7) from the image-related data (14) based on a calculation model (18).

## Description

The present invention relates to a method for operating a clarification setup of a bioprocess installation according to the general part of claim 1, to a clarification setup according to claim 15 and to a use of a centrifuge chamber for performing said method according to claim 16.

The term "bioprocess" presently represents any kind of biotechnological process, in particular a biopharmaceutical process. An example of such a bioprocess is the use of a bioreactor to cultivate microorganisms or mammalian cells under given conditions, wherein a cell broth is transferred from the bioreactor to a downstream process. The term "bioreactor" in this context means any manufactured device or system that supports a biological environment by allowing monitoring and control of at least one parameter. The term "bioproduct" presently represents any kind of compound produced in such a bioprocess. Examples for such bioproducts are proteins, in particular antibodies, growth factors or hormones, metabolites or any other molecule as well as cells or cellular components, such as organelles.

The method in question for operating a clarification setup may be applied in various fields of biotechnology. High efficiency in this field has been driven by the increasing demand for bioproducts, such as biopharmaceutical drugs. The efficiency in this sense is regarding not only the cost-effectiveness of the components to be used but also the controllability of the processes connected thereto. The method in question relies on optimal centrifuge chamber filling level determination to optimize controllability as well as time-effectiveness of the bioprocess.

A known method for operating a clarification setup (EP 2 310 486 B1) comprises a fluidized bed centrifuge with a number of centrifuge chambers, which are each assigned a chamber inlet and a chamber outlet. The clarification setup also comprises a pumping arrangement assigned to the fluidized bed centrifuge and a liquid network for the transport of the liquid, which is the cell broth to be clarified. Finally, the clarification setup comprises an electronic process control for controlling at least the fluidized bed centrifuge and the pumping arrangement.

To accurately determine particle filling levels of centrifuge chambers in fluidized bed centrifugation processes, it is currently necessary to provide at least the fluid flow throughput as well as the cell suspension characteristics, such as cell diameter, cell concentration and the like in order to calculate a respective particle filling level at a given point in time.

The term "particle" is to be understood in a broad sense and means any particulate solid compound, in particular cells, cell debris, proteins, (magnetic) microbeads or the like, which may be of biological or non-biological origin.

The term "particle filling level" is to be understood in a broad sense. The particle filling level is a measure for the filling of centrifuge chambers with particles and can be reflected by a percentage particle filling of the centrifuge chamber, a specific particle mass, volume, concentration or the like. Moreover, the particle filling level can be represented by a specific particle filling level at a given time or as a change in particle filling level over time, thereby also being reflective of the particle filling rate.

However, this serves only as an approximation and in case the filling level is underestimated in a fluidized bed centrifugation process, the system accidentally overfills and the fluidized bed centrifugation process becomes inefficient, since cells might be introduced into the following downstream process. On the other hand, in case the filling level is overestimated, the fluidized bed centrifugation process is inefficient due to a higher number of required centrifugation cycles to process a specific volume, such as a specific bioreactor volume.

The controllability within the known method is somewhat compromised, as the calculation of the filling level is time consuming and not even very accurate. With this, the efficiency of the overall process and the reproducibility each may be brought only to a certain level.

Another known method ("Operation and Maintenance Manual kSep400", Sartorius Stedim Biotech), which is the starting point for the invention, relies on the production of monitoring sensor data from a camera sensor, which monitoring sensor data are being analyzed manually by a user. Based on the experience of this user, those monitoring sensor data are being utilized to operate the clarification setup. Again, this method is time consuming and the reproducibility is restricted.

It is therefore an object of the present invention to improve the known method for operating a clarification setup such that the bioprocess efficiency and reproducibility is improved with as little effort as possible.

The above noted problem is solved by a method for operating a clarification setup according to the general part of claim 1 with the features of the characterizing part of claim 1.

Due to the floating state of the particles within the centrifuge chamber, the particle accumulation is proceeding in an ordered and reproducible way. In a wide range, this effect is taking place independently from the media to be processed. In particular it has been found that this leads to a visible phase boundary, which, with ongoing particle agglomeration, is travelling across the centrifuge chamber, wherein the location of the phase boundary represents the objective filling level of the centrifuge chamber. It is particularly interesting that it has been realized that, in contrast to known non-fluidized bed centrifuges, the proposed invention leads to a visible phase boundary without exposing the centrifuged cells to such high acceleration forces. Such high acceleration forces could otherwise lead to cell damage and even cell death. Hence, the cells are here separated under milder conditions, leading to a higher viability, which is crucial in case the cells are to be re-used in a subsequent bioprocess. Additionally, with the proposed invention, the cell number within a centrifuge chamber can be estimated more accurately. This is due to the fact that particle agglomeration under non-fluidized bed conditions is fundamentally different, since the acceleration forces acting on the cells are much higher. This is why in the proposed solution the cells in the particle agglomeration are not as densely packed as they would be under non-fluidized bed conditions. Thereby, the particle filling level can be calculated more precisely. Also, this is particularly advantageous for a more precise calculation of the required cell number that is necessary in order to inoculate a potential subsequent bioprocess. Moreover, it has been found, that the above noted, systematic particle agglomeration allows for the systematic calculation of the filling level based on a calculation model, which may even be performed in real time.

In detail, it is proposed, that in a monitoring routine, image-related data representing an optical image of the centrifuge chamber content are being produced by the monitoring sensor arrangement and a particle filling level is being calculated by the electronic process control from the image-related data based on a calculation model.

The achievement of the invention is an automatic, camera-based determination of the filling level, preferably in real time, to accelerate the production process in an organism-independent, cost-effective and non-invasive way. The determination of the centrifuge chamber load in real time reduces unnecessary waiting times, minimizes the number of centrifugation cycles necessary to process the bioreactor volume and prevents particle breakthrough of valuable cells. The term "particle breakthrough" relates to the point in time during the fluidized bed centrifugation process, when the at least one centrifuge chamber is filled to capacity with particles, preferably cells, and wherein a continued loading of the centrifuge chamber leads to an overfilling of said centrifuge chamber.

By using automatic filling level determination via image processing, an accurate signal for each chamber can be acquired. As an image-related approach, the determined filling level is independent from broth composition, such as the amount of viable cells, cell types, and cell diameter, and is even able to reflect the presence of impurities or dead cells.

Claims 2 and 3 are directed to preferred embodiments of the calculation model used in the monitoring routine. According to these embodiments, different optical properties or phase boundaries within a centrifuge chamber are used to solve the problem of determining a particle filling level in an organism-independent way.

In claim 4, especially preferred embodiments regarding translucent properties of the centrifuge chamber are specified. Such a design allows for a determination of the particle filling level in a non-invasive manner. In particular, due to the translucent properties, a window or the like does not have to be provided in each centrifuge chamber.

The preferred embodiments according to claims 5 and 6 refer to the different design and arrangement options for the camera unit as the at least one monitoring sensor of the monitoring sensor arrangement, which enables an increased flexibility regarding the choice of the camera as well as its respective arrangement.

In claims 7 and 8, especially preferred embodiments regarding the definition and calculation of the particle filling levels are specified. These specifications allow for an optimized and simplified determination of the particle filling levels.

Claims 9 and 10 are directed to preferred embodiments regarding a calibration sensor arrangement and options for a calibration routine. These features are particularly advantageous to calibrate the monitoring sensor arrangement, hence improving the accuracy of the calculation model as well as the determination of the absolute maximum particle filling level.

According to the especially preferred embodiment of the first alternative of claim 11, at least one parameter of the clarification setup is being adjusted by the electronic process control based on the particle filling levels, enabling a uniformly filling of each centrifuge chamber. This allows for a simultaneous discharge of all centrifuge chambers, hence enabling a more efficient usage of the centrifuge chamber capacities with low effort.

The preferred embodiment according to the second alternative of claim 11 refers to a decision point, based on which the electronic process control initiates centrifugation cycles.

According to the preferred embodiments of claim 12, nominal maximum particle filling levels and/or a nominal zero particle filling levels is/are stored in the electronic process control, defining an offset each to prevent particle breakthrough as well as unnecessary waiting times with high efficiency.

The preferred embodiment according to claim 13 is directed to the presence of a downstream setup in the bioprocess installation and that the electronic process control adapts at least one parameter of the downstream setup in an adapting routine based on a predefined adapting strategy. This is particularly advantageous since it enables an increased flexibility regarding the choice of the subsequent downstream unit as well as downstream method with reduced waiting times. Thereby, the subsequent bioproduct purification can be individually customized to the specific bioproduct needs, leading to increased bioprocess efficiency and reduced process costs with high flexibility.

Claim 14 is directed to a preferred embodiment, wherein in an analyzing routine the presence of contaminants is detected. These specifications allow for an additional level of information, helping to distinguish between living and dead cells as well as contaminants, which is fundamental for example in order to determine the required cell number to be re-used for a subsequent bioprocess.

According to a second independent teaching according to claim 15, the clarification setup for performing the proposed method is claimed as such. All explanations given for the proposed method are fully applicable to the proposed clarification setup.

A third independent teaching according to claim 16 is directed to the use of a proposed centrifuge chamber for performing the proposed method. All explanations given before are fully applicable to the proposed centrifuge chamber.

A fourth teaching, that may be claimed independently, is directed to an electronic process control. The electronic process control is designed to perform the proposed method according to any one of the preceding claims. Again, all explanations given before are fully applicable to this proposed fourth teaching. The electronic process control preferably comprises a data processing system for the realization of the proposed method.

A fifth teaching, that may be claimed independently as well, is directed to a computer program product for the proposed electronic process control. The computer program product is configured to realize the proposed method, in particular, to realize the above-noted routines. Again, all explanations given for the proposed method are fully applicable to the proposed computer program product.

A sixth teaching, that may be claimed independently as well, is directed to a computer-readable storage media, on which the computer program is stored. Again, all explanations given for the proposed method are fully applicable to the proposed readable storage media.

In the following, an embodiment of the invention is explained with respect to the drawings. The drawings show in
- Fig. 1: schematically a preferred embodiment of a proposed bioprocess installation, with which a proposed method is executable,
- Fig. 2: perspectively the working principle of the proposed method according to Fig. 1,
- Fig. 3: a flow chart representing the preferred core working principle of the proposed method according to Fig. 1.

The proposed method for operating a clarification setup 1 of a bioprocess installation 2 is preferably assigned to the upstream and downstream processes of a bioprocess, processing a liquid, in particular a cell broth for cell cultivation and/or bioproduction.

The term "liquid" is to be understood in a broad sense. It includes not only a pure liquid as such, but also emulsions and suspensions, e.g. a heterogeneous mixture of at least two different liquids or a heterogeneous mixture of solid particles and liquid.

The term "cell broth" is a suspension of particles in a solvent, in particular cells and/or cell debris in media. It describes in particular the entirety of the cultivation medium and the respective organism cultured in the cultivation medium.

The term "upstream process" involves all the steps related to cell bank, inoculum (seed train) development, media development, optimization of growth kinetics and the cultivation process itself as well as the corresponding in-process control. The harvest of cells can be seen as both, part of upstream- and part of downstream processing. The term "downstream process" involves all the steps related to the recovery and the purification of bioproducts, particularly biopharmaceuticals, from natural sources such as animal or plant tissue or cell broth, including the recycling of salvageable components and the proper treatment and disposal of waste.

In general, the cultivation of cells is currently used for the production of biopharmaceuticals, in particular proteins, such as human insulin, growth factors, hormones, vaccines, or antibodies, antibody derivatives, or the like. The bioproducts may as well be non-biopharmaceuticals, such as enzymes for food processing, laundry detergent enzymes, biodegradable plastics or biofuels. The focus of the present invention is on biopharmaceutical products secreted by the cells into the supernatant, such as antibodies or exosomes. Additionally or alternatively, the product can be the cells themselves, in particular mammalian cells including stem cells or immune cells such as CAR-T cells for the treatment of cancer.

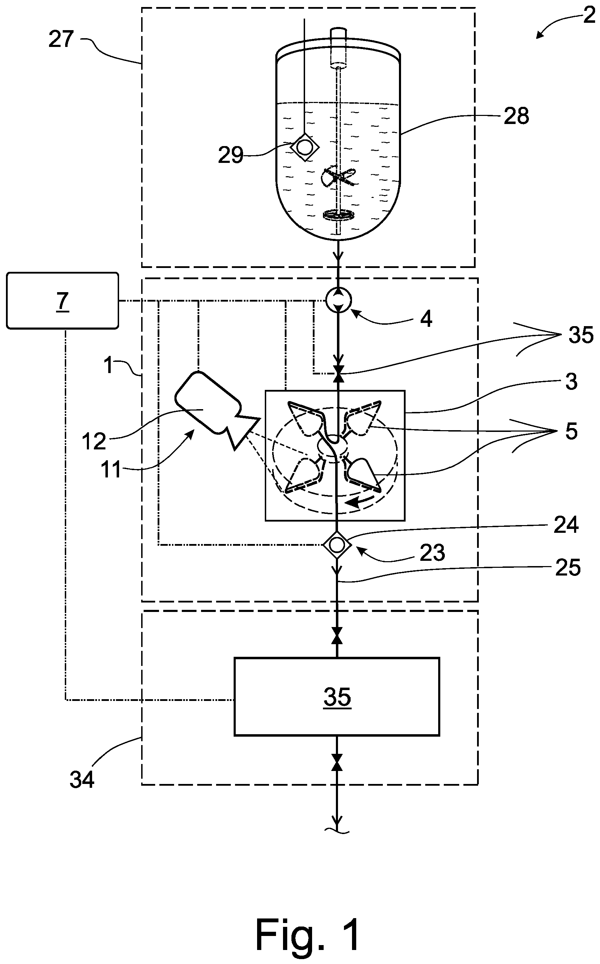

As shown in Fig. 1 to 3, according to all embodiments, the proposed method for operating a clarification setup 1 of a bioprocess installation 2 employs at least one fluidized bed centrifuge 3 for the clarification of a cell broth by centrifugation and a pumping arrangement 4 assigned to the fluidized bed centrifuge 3. The pumping arrangement 4 comprises at least one pump. The fluidized bed centrifuge 3 comprises at least one centrifuge chamber 5, preferably an even number of centrifuge chambers 5, further preferably exactly four centrifuge chambers 5, which are being turned around a geometrical centrifuge axis 6 and develop a fluidized bed during normal operation. Preferably, each centrifuge chamber 5 comprises at least one assigned pump for pumping liquid in or out of the chamber. The bioprocess installation 2 further comprises an electronic process control 7 for controlling at least the fluidized bed centrifuge 3 and the pumping arrangement 4.

"Centrifugation" is a term for sedimentation of particles in an artificially, by centrifugal forces created, gravitational field, wherein a significant reduction of separation time is achieved via large accelerating forces.

Here, the centrifuge is designed as a fluidized bed centrifuge 3 for performing a continuous centrifugation process. Preferred setups of the fluidized bed centrifuge 3 are described in EP 2 485 846 A1, the contents of which are hereby incorporated by reference herein.

The fluidized bed centrifuge 3 comprises a rotor with the at least one centrifuge chamber 5 attached thereto, which may be rotated around the centrifuge axis 6 by a, preferably electric, motor. The centrifuge revolution speed and the pumping rate are adjustable by the electronic process control 7, with the aim to establish a fluidized bed of particles, such as cells or cell debris, in the fluidized bed centrifuge 3. A fluidized bed is achieved when the centrifugal force on a particle is equal to the opposing fluid flow force so that a zero net force is exerted on the particle.

According to Fig. 1, the cell broth is being led through the fluidized bed centrifuge 3. The fluidized bed centrifuge 3 is being operated in a forward operation for a loading cycle 8 and/or a washing cycle 9 and in a backward operation for a particle discharging cycle 10.

"Forward operation" means one out of two possible fluid flow directions in a fluidized bed centrifuge and describes the operation leading to a separation of liquid and solid particles, such as media and cells. This "forward operation" allows, on the one hand, a washing of separated cells with buffer or media, preferably cultivation media, further preferably enriched media, and/or, on the other hand, the clarification of the cell broth. The goal here is to clarify the liquid supernatant from solid particles such as cells, cell debris, etc., which solid particles are considered biomass. The product to be obtained in this forward operation is the supernatant of the cell broth containing a bioproduct of interest, e.g., a recombinant protein, in particular an antibody.

The term "enriched media" describes media that comprise higher concentrations of vitamins, growth factors, trace nutrients, as well as carbon-source, nitrogen-source and/or amino acid concentrations or the like and, preferably, allow the respective organism to grow at its maximum growth rate due to the optimized nutrient concentrations. Growth factors and trace nutrients are included in the media for organisms incapable of producing all of the vitamins they require themselves. Inorganic nutrients, including trace elements such as iron, zinc, copper, manganese, molybdenum and cobalt are typically present in unrefined carbon and nitrogen sources but may have to be added when purified carbon and nitrogen sources are used.

The loading cycle refers to a cycle of loading the respective centrifuge chamber 5 in forward operation of the fluidized bed centrifuge 3 with cell broth to be centrifuged. Hence, during the loading cycle 8, cell broth loaded into the centrifuge chamber 5 proceeds to form a growing particle accumulation in the centrifuge chamber 5.

The washing cycle refers to a cycle of washing the respective centrifuge chamber 5 in forward operation of the fluidized bed centrifuge 3 with media or buffer. This washing cycle preferably serves for a supply of fresh nutrients to the cells.

Alternatively, the fluidized bed centrifuge 3 can be operated in a backward operation. "Backward operation" means the second out of two possible fluid flow directions in the fluidized bed centrifuge 3 and describes the operation leading to a discharge of the separated solid particles, preferably cells. The product to be obtained in backward operation are the cells in the cell broth.

Hence, the discharging cycle refers to the cycle assigned to the fluidized bed centrifuge 3, wherein in backward operation the centrifuge chamber 5 is drained from solid particles, preferably cells. This discharging cycle 10 can i.a. serve for a re-use of cells in a subsequent bioprocess.

Moreover, the clarification setup 1 comprises a monitoring sensor arrangement 11 with at least one optical sensor 12 for producing monitoring sensor data, which are being transmitted to the electronic process control 7. The optical sensor 12 is directed to the centrifuge chamber 5, as is shown in Fig. 1.

It is particularly essential for the invention, that in a monitoring routine 13, monitoring sensor data in the form of image-related data 14 representing an optical image 15 of the centrifuge chamber content 16 are being produced by the monitoring sensor arrangement 11. From these image-related data 14, the electronic process control 7 calculates a particle filling level 17 based on a calculation model 18.

The calculation model 18 is to be understood as a rule system for the calculation of the filling level 17 based on the image related data 14, as will be explained later. In a preferred embodiment, the calculation model 18 may be exchanged between two different operating instances or even within one and the same operating instance. Moreover, the calculation model is highly adaptable to different bioprocess settings leading to altered optical properties, such as regarding the choice of the used cultivation media, which can lead to different contrasts, different brightnesses, different densities or the like. According to another preferred embodiment, the calculation model can be adapted to different cell types comprising different particle sizes, different shapes, different concentrations or the like. This adaptability renders the proposed method exceptionally flexible.

The term "image-related data" is to be understood in a broad sense. It represents at least one image and can in this sense be a normal photographic representation. In addition, the term "image-related data" may comprise monitoring sensor data of other sensors of the monitoring sensor arrangement 11 regarding other properties of the liquid. Such properties may, for example, be flow rate, flow velocity, cell type, carbon-source concentration, nitrogen-source concentration, amino acid concentration, pH, temperature, oxygen concentration, carbon dioxide concentration, conductivity, pressure, DNA concentration, protein concentration or biomass concentration.

The monitoring routine 13 or any other proposed routine are preferably initiated according to a common predefined strategy or individual, predefined strategies. For example, the monitoring routine 13 may generally be initiated, when the loading cycle 8 is initiated. However, it may even be more effective to have the monitoring routine 13 initiated not before a certain amount of particle agglomeration has taken place, for example after a predefined amount of loading time.

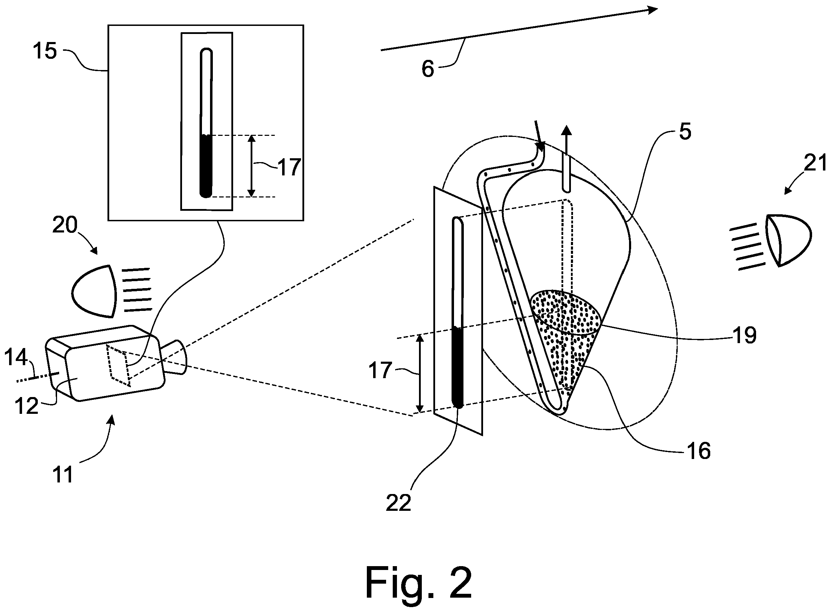

In the embodiment according to Fig. 2, here and preferably, in the monitoring routine 13, according to the calculation model 18, the particle filling level 17 is being calculated based on the different optical properties of the centrifuge chamber content 16. These different optical properties are in particular a different translucence, different colour and/or different brightness, of the centrifuge chamber content 16 within and outside the particle accumulation. This may for instance be a different colour of the solid particles, preferably cells, caused by characteristics that are specific to a certain particle type, preferably cell type, such as a different turbidity, a colour-, brightness- and/or density-difference within and outside the particle accumulation.

The term "particle accumulation" means here the mass of accumulated particles within a centrifuge chamber 5.

In a preferred embodiment, in the monitoring routine 13, according to the calculation model 18, the phase boundary 19 between the particle accumulation and the particle free rest of the centrifuge chamber content 16 is detected in the optical image 15. Such a phase boundary 19 may develop due to differences in the composition of the particle accumulation and the particle free rest of the centrifuge chamber content 16, such as a difference in density, a difference in affinity, e.g. hydrophilic or hydrophobic properties, a difference in colour, translucence and/or brightness. Subsequently, the particle filling level 17 is being calculated from the position of the phase boundary 19 based on the calculation model 18. The phase boundary may be detected by image processing, for example by an algorithm for line recognition. It may also be detected simply by determining two areas of different optical properties, such as contrast, brightness or the like within the optical image 15 representing the centrifuge chamber content 16. These different optical properties preferably represent the particle agglomeration on the one hand (e.g. comprising a lower brightness) and the particle free rest (e.g. comprising a higher brightness) of the centrifuge chamber content 16 on the other hand.

According to another calculation model 18, the filling level 17 is calculated by applying a correlation function to the image-related data 14, which results in an indication regarding the relation in sizes between the area of the particle agglomeration and the area of the particle free rest of the centrifuge chamber content 16 in the respective optical image 15.

In another preferred embodiment according to Fig. 2, the centrifuge chamber 5 as such is made of a translucent material. Preferably, the centrifuge chamber 5 is designed as single use component, preferably made of a translucent, biocompatible plastic or bioplastic material, such as PE, PP, PS, PVC, PET, PUR or the like. Hence, the monitoring sensor arrangement 11 is detecting the optical image 15 of the centrifuge chamber content 16 through the translucent material of the centrifuge chamber 5. This is not only cost efficient, as a viewing window for the optical sensor 12 does not have to be introduced in each centrifuge chamber 5, but also extremely flexible, as different areas of the centrifuge chamber content 16 may be viewed by the optical sensor 12 without changing the setup.

According to another preferred embodiment, as can be seen in Fig. 1 and Fig. 2, the at least one optical sensor 12 of the monitoring sensor arrangement 11 is a camera unit, preferably a 2D or 3D camera. Exemplary 2D cameras to be used are two-dimensional CCD-array sensors used for video cameras and digital cameras as well as CMOS-sensors used for smart phones and tablets. The optical sensor 12 is preferably located inside or outside the fluidized bed centrifuge 3, further preferably, inside or outside the centrifuge rotor chamber.

The viewing direction of the optical sensor 12, here and preferably the camera unit is preferably basically parallel to the geometrical centrifuge axis 6. It may, however, be inclined, which may be advantageous in terms of the optimized use of the available space. The term "basically parallel" means here no ideal parallel line in a mathematical sense, but in a colloquial sense, wherein the camera unit is for the better part arranged parallel (see Fig. 2) to the geometrical centrifuge axis 6.

In Fig. 2, two alternatives for the light arrangement 20, 21 are displayed. In the first alternative, the light of the light arrangement 21 is shining through the centrifuge chamber content 16 and onto the monitoring sensor arrangement 11. This improves the reproducibility of the measurements by the optical sensor 12, independently from any other surrounding conditions. In this preferred alternative, the light arrangement 21 is positioned basically opposite of the monitoring sensor arrangement 11. The term "basically opposite" means here no ideal opposite arrangement in a mathematical sense, but in a colloquial sense, wherein the light arrangement 21 is for the better part arranged opposite of the monitoring sensor arrangement 11. This means that the cell broth loaded in the centrifuge chamber 5 is being penetrated by the light of the light arrangement 21, which supports a clear detection of the area of particle agg lom eration.

In an alternate embodiment included in Fig. 2, the light of the light arrangement 20 is shining onto the centrifuge chamber content 16 and is being reflected onto the monitoring sensor arrangement 11. The reflection is preferably realized by a reflecting element, such as a mirror, a reflector or the like. The light source of the light arrangement 20, 21 is preferably at least one LED, further preferably at least one stroboscopic LED.

Preferably, the monitoring sensor arrangement 11 and/or the light arrangement 20, 21 is/are being synchronized with the turning of the at least one centrifuge chamber 5. The term "synchronized" means the bidirectional temporal coordination of events to operate a system in unison.

According to an especially preferred embodiment, a stroboscopic LED or an array of LED's and/or the camera unit are synchronized with the turning of the at least one centrifuge chamber 5. In particular, the synchronized operation of the monitoring sensor arrangement 11 makes sure to fade out any optical information, which is not related to the centrifuge chamber 5 itself.

In another preferred embodiment according to Fig. 2, the particle filling level 17 represents the range between a particle free state of the centrifuge chamber 5 and a state of maximum particle accumulation. This state of maximum particle accumulation is defined by the state, which is the borderline to particle breakthrough and which is followed by particle breakthrough when proceeding with the loading cycle 8. According to an especially preferred embodiment, the state of maximum particle accumulation corresponds to the absolute maximum particle filling level, and/or, the particle free state of the centrifuge chamber 5 corresponds to the absolute zero particle filling level. The absolute zero particle filling level corresponds to the centrifuge chamber 5 being completely empty of particles, in particular cells. The absolute maximum particle filling level corresponds to the maximum particle filling level just before the particle breakthrough during the loading cycle 8.

According to another preferred embodiment shown in Fig. 2, the fluidized bed centrifuge 3 comprises a monitoring aperture 22 in a monitoring panel, through which the centrifuge chamber contents 16 may be monitored. The monitoring aperture 22 is assigned and aligned to the optical sensor 12. It is preferred that the fluidized bed centrifuge 3 comprises exactly one monitoring aperture 22 in a monitoring panel for monitoring the centrifuge chamber contents 16 of all the centrifuge chambers 5. In this case, the monitoring panel with the monitoring aperture 22 is preferably fixed, while the centrifuge chamber 5 is turning around the centrifuge axis 6.

Alternatively, according to an especially preferred embodiment, the fluidized bed centrifuge 3 comprises one monitoring aperture 22 in a monitoring panel for each centrifuge chamber 5, wherein each monitoring aperture 22 is aligned to the assigned centrifuge chamber 5. In this preferred embodiment, each monitoring aperture 22 in the respective monitoring panel is moving with the respective centrifuge chamber 5 around the centrifuge axis 6.

Here and preferably, in the monitoring routine 13, according to the calculation model 18, the particle filling level 17 is being calculated from the distribution of brightness and/or colours over the monitoring aperture 22 based on the calculation model 18.

It is especially preferred that the monitoring aperture 22 extends along a radial direction with respect to the geometrical centrifuge axis 6. The realization of such a monitoring aperture 22 reduces the image processing to the monitoring aperture 22 and therefore simplifies the image processing considerably. In particular, detecting the phase boundary 19 within the monitoring aperture 22 is possible with low technical effort.

In another embodiment, according to Fig. 1, the clarification setup 1 comprises a calibration sensor arrangement 23 with at least one calibration sensor 24 for producing calibration sensor data, which are being transmitted to the electronic process control 7. Here and preferably, the calibration sensor arrangement 23 detects the presence of particles within a liquid line 25 downstream of the fluidized bed centrifuge 3. Additionally or alternatively, the calibration sensor arrangement 23 detects the flow rate of particles within a liquid line 25 downstream of the fluidized bed centrifuge 3. These detections are being preferably conducted during the loading cycle 8 and/or the washing cycle 9. Therefore, the at least one calibration sensor 24 is preferably designed as a particle sensor, further preferably as a cell detecting sensor, and/or a flow rate sensor.

Preferably, according to Fig. 1, in a calibration routine, the monitoring sensor data of the monitoring sensor arrangement 11 are being correlated with the calibration sensor data of the calibration sensor arrangement 23, in order to improve the accuracy of the calculation model 18. For instance, the monitoring sensor arrangement 11 detects a specific particle filling level 17, which is then correlated with the calibration sensor data. In case the calibration sensor arrangement 23 detects the absence of particles, preferably cells, the correlation gives the information that no particle breakthrough occurred. Additionally or alternatively, the absolute maximum particle filling level is being derived.

Additionally or alternatively, in a calibration routine, which may be the above calibration routine or another calibration routine, a loading cycle 8 proceeds to and beyond the point of particle breakthrough. The particle filling level 17 calculated at the point of particle breakthrough is being stored as the absolute maximum particle filling level in the electronic process control 7. In this scenario, in case the calibration sensor arrangement 23 detects the presence and/or flow rate of particles, preferably cells, this gives the information that a particle breakthrough actually occurred. This specific particle filling level 17 is then saved in the electronic process control 7 as the absolute maximum particle filling level.

According to an especially preferred embodiment, in an adjusting routine, at least one parameter of the clarification setup 1 is being adjusted by the electronic process control 7 based on the particle filling levels 17 of the centrifuge chambers 5 according to a predefined adjusting strategy. The at least one adjusted parameter of the clarification setup 1 is preferably the centrifugation velocity of the rotor of the fluidized bed centrifuge 3 and/or the volumetric flow rate of at least one pump, preferably of all pumps of the pumping arrangement 4 that are assigned to a centrifuge chamber 5.

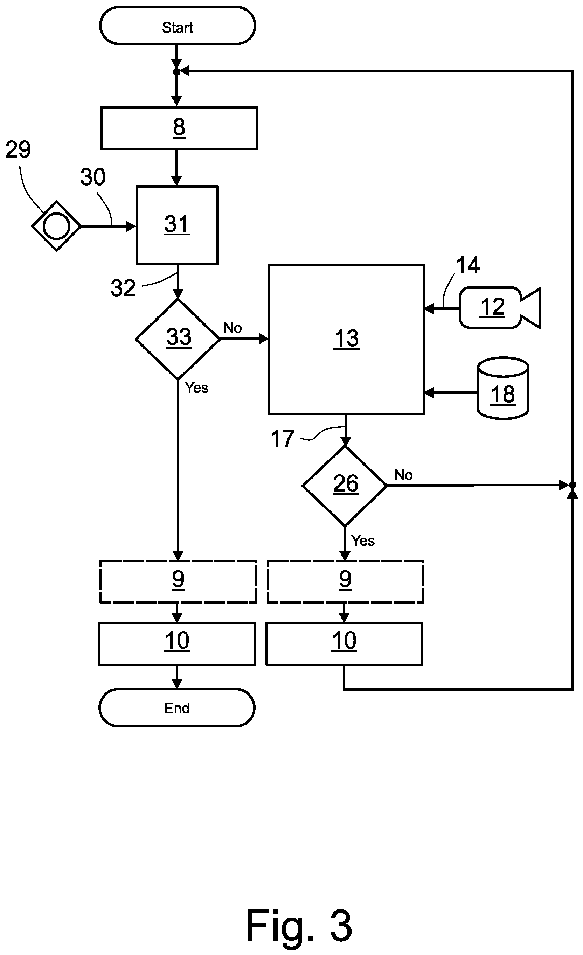

In another preferred embodiment, according to Fig. 3, based on a decision point 26, the electronic process control 7 initiates centrifugation cycles according to a predefined automation strategy based on the calculated particle filling level 17 in an automation routine. The fluidized bed centrifuge 3 is being operated in such "centrifugation cycles" comprising loading, washing and discharging cycles.

For instance, according to an above noted, predefined automation strategy based on the calculated particle filling level 17, when the monitoring sensor arrangement 11 detects that the measured particle filling level 17 in a centrifuge chamber 5 corresponds to the absolute maximum particle filling level, the electronic process control 7 initiates the washing cycle and/or discharging cycle, in order to prevent particle breakthrough (Fig. 3). According to another example, in case that the measured particle filling level 17 in a centrifuge chamber 5 corresponds to the absolute zero particle filling level, the electronic process control 7 initiates the centrifugation loading cycle 8, in order to prevent unnecessary waiting times.

In another especially preferred embodiment, according to Fig. 1, the bioprocess installation 2 comprises a cultivation setup 27 with at least one upstream unit 28 for producing the bioproduct, in particular a bioreactor. In general, when using continuous upstream processes, such as perfusion cultivation, the cell broth level in the upstream unit 28 is preferably static, hence being able to provide a continuous liquid stream containing cells and/or product to the fluidized bed centrifuge 3. However, in particular in case a continuous upstream process is stopped or discontinuous upstream processes are used, such as batch or fed-batch processes, it is preferred that the upstream unit 28 comprises at least a filling level sensor 29 for generating filling level sensor data 30 of the upstream unit 28.

The filling level sensor 29 is set up to detect a cell broth filling level within the upstream unit 28. Thereby, the harvesting of the upstream unit 28 can be terminated automatically by the electronic process control 7, once the filling level falls below a predefined lower filling level threshold. The filling level sensor 29 might be any sensor for determining the presence of cell broth qualitatively and/or quantitatively, such as a capacitance sensor, an optical sensor, a bubble sensor, or the like.

According to Fig. 3, in an information retrieval 31 the electronic process control 7 retrieves at least a biomass status 32 of the upstream unit 28 from the filling level sensor data 30 produced by the filling level sensor 29. Thereby, the biomass status 32 indicates, whether or not there is still cell broth present in the upstream unit 28 to be processed. Subsequently, the electronic process control 7 initiates the monitoring routine 13, or, the washing cycle 9 and/or the discharging cycle 10 based on the derived biomass status 32.

Here and preferably, as can be seen in Fig. 3, in an information retrieval 31 the electronic process control 7 retrieves the biomass status 32 of the upstream unit 28 to determine, based on the decision point 33, whether or not the cell broth has been completely processed yet. According to the retrieved information, in case the cell broth has been completely processed (see Fig. 3 "Yes"), the electronic process control 7 initiates the washing cycle 9 and/or the discharging cycle 10.

However, in case the cell broth has not been completely processed yet (see Fig. 3 "No"), the electronic process control 7 proceeds with the monitoring routine 13.

Additionally or alternatively, and according to an especially preferred embodiment, the upstream unit 28 comprises a biomass sensor for generating biomass sensor data of the upstream unit 28. The biomass sensor might be any sensor for determining the presence of biomass in the upstream unit 28 qualitatively and/or quantitatively, such as a capacitance sensor, an optical sensor, or the like. In case the biomass status 32, retrieved in an information retrieval 31 by the electronic process control 7 of the upstream unit 28, reaches a predefined biomass threshold, such as a cell density threshold, the electronic process control 7 initiates the loading cycle 8. Thereby, the harvesting of the upstream unit 28 can be initiated automatically, once a predefined lower biomass threshold has been exceeded, which is particularly advantageous for fed-batch or perfusion processes, where cells are preferably kept in a proliferating state. This automation approach, employing the filling level sensor 29 and/or the biomass sensor, renders the proposed method exceptionally flexible.

Preferably, a nominal maximum particle filling level is stored in the electronic process control 7. This nominal maximum particle filling level is preferably below the absolute maximum particle filling level by a predefined upper offset. This upper offset is preferably up to 25 %, further preferably up to 10 %, further preferably up to 5 %, further preferably up to 1 % of the total centrifuge chamber volume. In the decision point 26, when the nominal maximum particle filling level is approached during the loading cycle 8, according to the automation strategy, the loading cycle 8 is terminated and, preferably, the washing cycle 9 or the discharging cycle 10 is initiated by the electronic process control 7.

Additionally or alternatively, a nominal zero particle filling level is stored in the electronic process control 7. This nominal zero particle filling level is above the absolute zero particle filling level by a predefined lower offset. This lower offset is preferably up to 25 %, further preferably up to 10 %, further preferably up to 5 %, further preferably up to 1 % of the total centrifuge chamber volume. Based on the decision point 26, when the nominal zero particle filling level is approached during the discharging cycle 10, according to the automation strategy, the discharging cycle 10 is terminated and, preferably, the loading cycle 8 is initiated.

As indicated by Fig. 1, the bioprocess installation 2 comprises a downstream setup 34 with at least one downstream unit 35. This downstream setup 34 is preferably at least one out of the group of filtration setup, viral inactivation setup, chromatography setup and/or viral filtration setup. The at least one downstream unit 35 is preferably at least one out of the group of microfiltration unit, ultrafiltration unit, capture chromatography unit, viral inactivation unit, diafiltration unit, intermediate (purification) chromatography unit, polishing chromatography unit, viral filtration unit and/or sterile filtration unit.

In an adapting routine, the electronic process control 7 adapts at least one parameter of the downstream setup 34 according to the image-related data 14 based on a predefined adapting strategy. According to an especially preferred embodiment, and to be understood just as an example, this at least one individually adaptable parameter is preferably the choice of the type of chromatography column, the required column volume, the required flow rate, the required stationary phase of the chromatography column, and/or, the choice of the type of filter, the required filter pore size, and/or, the choice of pH for viral inactivation, the duration of viral inactivation, or the like.

Preferred types of chromatography columns are affinity chromatography, in particular Protein A affinity chromatography, ion-exchange chromatography (IEX), such as anion exchange chromatography (AEX) or cation exchange chromatography (CEX), hydrophobic interaction chromatography (HIC), size-exclusion chromatography (SEC) or any other type of chromatograph. Moreover, these chromatography types can be operated in axial flow or radial flow. The at least one downstream unit 35 comprises at least one chromatography column, preferably a multitude of chromatography columns, set up for multi-column simulated moving bed (SMB) chromatography.

According to another preferred embodiment, in an analyzing routine, an irregularity state attributed to the presence of impurities, preferably contaminating organisms, is detected based on predefined irregularity features in the optical image 15.

The irregularity features in the optical image 15 go along with characteristic turbidity within the liquid, which may easily be detected by the camera unit as optical contrasts and/or aggregation of particles, particle clouds, change in colour and/or brightness or the like. Preferably, the attributed irregularity state is "contam inated".

Preferably, at least one component of the clarification setup 1, in particular the fluidized bed centrifuge 3, the centrifuge chambers 5, the upstream unit 28 and/or the downstream setup 34, further preferably all components of the bioprocess installation 2, are designed as single-use components.

According to another, independent teaching, a clarification setup 1 of a bioprocess installation 2 for performing the above noted method is claimed as such, with a fluidized bed centrifuge 3 for the clarification of a cell broth by centrifugation and a pumping arrangement 4 assigned to the fluidized bed centrifuge 3. The fluidized bed centrifuge 3 comprises at least one centrifuge chamber 5, which is being turned around a geometrical centrifuge axis 6. Moreover, the bioprocess installation 2 comprises an electronic process control 7 for controlling at least the fluidized bed centrifuge 3 and the pumping arrangement 4. The fluidized bed centrifuge 3 is being operated in a forward operation for a loading cycle 8 and/or a particle washing cycle 9 and in a backward operation for a particle discharging cycle 10. Here and preferably, during the loading cycle 8, cell broth loaded into the centrifuge chamber 5 proceeds to form a growing particle accumulation in the centrifuge chamber 5. The clarification setup 1 comprises a monitoring sensor arrangement 11 with at least one optical sensor 12 for producing monitoring sensor data, which are being transmitted to the electronic process control 7. Reference is made to all explanations given before

It is essential, that in a monitoring routine 13, image-related data 14 representing an optical image 15 of the centrifuge chamber content 16 are being produced by the monitoring sensor arrangement 11 and a particle filling level 17 is being calculated from the image-related data 14 by the electronic process control 7.

According to another, independent teaching, the use of a centrifuge chamber 5, which as such is made of a translucent material, for performing the above noted method is claimed as such. Reference is made to all explanations given before.

The electronic process control 7 of the bioprocess installation 2 for performing the claimed method may be claimed as such as well. Again, reference is made to all explanations given before.

It is essential, that the electronic process control 7 is designed for performing the proposed method by controlling at least the fluidized bed centrifuge 3 and the pumping arrangement 4.

Preferably, the electronic process control 7 is designed to perform the proposed method by controlling the upstream unit 28, the clarification setup 1 with its fluidized bed centrifuge 3 and/or the downstream setup 34 with its downstream unit 35. The electronic process control 7 may be realized as a central unit controlling all or at least most of the components of the bioprocess installation 2. The electronic process control 7 may also be realized in a decentralized structure, comprising a number of decentralized units. In some embodiments, the at least one electronic process control 7 directs the opening and closing of one or more valve(s) 35, the rotational speed of the rotor, either directly or via a motor, and/or the flow direction and/or velocity of the fluid and/or particles from an upstream unit 28, such as a bioreactor.

Such an electronic process control 7 comprises for instance at least one digital control unit (DCU) and/or at least one multi fermenter control system (MFCS), which comprises a local processor unit and a local data storage itself. The MFCS also provides a centralized process management system, dispatching requests to the digital control unit. Additionally or alternatively, such an electronic process control 7 comprises preferably a computer, and/or a server, and/or a smartphone or the like. Here and preferably, the electronic process control 7 is individually adjustable and/or programmable and/or comprises at least one microprocessor, on which software may be run. All explanations given before are fully applicable to this teaching.

Preferably, the electronic process control 7 comprises a data processing system for the realization of the above-noted method, preferably comprising a local data storage and a local processor unit.

Finally, independent teachings may be directed to a computer program product for the electronic process control 7 and to a computer-readable storage media, on which the computer program product is stored, preferably in a non-volatile manner.

## Claims

1. Method for operating a clarification setup (1) of a bioprocess installation (2), wherein the clarification setup (1) comprises a fluidized bed centrifuge (3) for the clarification of a cell broth by centrifugation and a pumping arrangement (4) assigned to the fluidized bed centrifuge (3), wherein the fluidized bed centrifuge (3) comprises at least one centrifuge chamber (5), which is being turned around a geometrical centrifuge axis (6), wherein the bioprocess installation (2) comprises an electronic process control (7) for controlling at least the fluidized bed centrifuge (3) and the pumping arrangement (4), wherein the fluidized bed centrifuge (3) is being operated in a forward operation for a particle loading cycle (8) and/or a particle washing cycle (9) and in a backward operation for a particle discharging cycle (10), wherein in the case where a particle loading cycle (8) is provided, during the particle loading cycle (8), cell broth loaded into the centrifuge chamber (5) proceeds to form a growing particle accumulation in the centrifuge chamber (5), wherein the clarification setup (1) comprises a monitoring sensor arrangement (11) with at least one optical sensor (12) for producing monitoring sensor data, which are being transmitted to the electronic process control (7),
**characterized in**
**that** in a monitoring routine (13), image-related data (14) representing an optical image (15) of the centrifuge chamber content (16) are being produced by the monitoring sensor arrangement (11) and a particle filling level (17) is being calculated by the electronic process control (7) from the image-related data (14) based on a calculation model (18).

2. Method according to claim 1, **characterized in that** in the monitoring routine (13), according to the calculation model (18), the particle filling level (17) is being calculated based on the different optical properties, in particular different translucence, different colour and/or different brightness, of the centrifuge chamber content (16) within and outside the particle accumulation.

3. Method according to claim 1 or 2, **characterized in that** in the monitoring routine (13), according to the calculation model (18), the phase boundary (19) between the particle accumulation and the particle free rest of the centrifuge chamber content (16) is detected in the optical image (15) and that the particle filling level (17) is being calculated from the position of the phase boundary (19) based on the calculation model (18).

4. Method according to any one of the preceding claims, **characterized in that** the centrifuge chamber (5) as such is made of a translucent material and that the monitoring sensor arrangement (11) is detecting the optical image (15) of the centrifuge chamber content (16) through the translucent material of the centrifuge chamber (5).

5. Method according to any one of the preceding claims, **characterized in that** the at least one optical sensor (12) of the monitoring sensor arrangement (11) is a camera unit, preferably a 2D or 3D camera, and/or, that the viewing direction of the camera unit is basically parallel or inclined to the geometrical centrifuge axis (6).

6. Method according to any one of the preceding claims, **characterized in that** a light arrangement (20, 21) is assigned to the monitoring sensor arrangement (11) with light shining through the centrifuge chamber content (16) and onto the monitoring sensor arrangement (11) or with light shining onto the centrifuge chamber content (16) and being reflected onto the monitoring sensor arrangement (11), and/or, that the monitoring sensor arrangement (11) and/or the light arrangement (20, 21) is being synchronized with the turning of the at least one centrifuge chamber (5).

7. Method according to any one of the preceding claims, **characterized in that** the particle filling level (17) represents the range between a particle free state of the centrifuge chamber (5) and a state of maximum particle accumulation, which is defined by the state, which is the borderline to particle breakthrough and which is followed by particle breakthrough when proceeding with the loading cycle (8), preferably, that the state of maximum particle accumulation corresponds to the absolute maximum particle filling level, and/or, that the particle free state of the centrifuge chamber (5) corresponds to the absolute zero particle filling level.

8. Method according to any one of the preceding claims, **characterized in that** the fluidized bed centrifuge (3) comprises a monitoring aperture (22) in a monitoring panel, through which the centrifuge chamber contents (16) may be monitored, and that in the monitoring routine (13), according to the calculation model (18), the particle filling level (17) is being calculated from the distribution of brightness and/or colours over the monitoring aperture (22) based on the calculation model (18), preferably, that the monitoring aperture (22) extends along a radial direction with respect to the geometrical centrifuge axis (6).

9. Method according to any one of the preceding claims, **characterized in that** the clarification setup (1) comprises a calibration sensor arrangement (23) with at least one calibration sensor (24) for producing calibration sensor data, which are being transmitted to the electronic process control (7), and that the calibration sensor arrangement (23) detects the presence and/or the flow rate of particles within a liquid line (25) downstream of the fluidized bed centrifuge (3), preferably during the loading cycle (8) and/or the washing cycle (9), preferably, that in a calibration routine, the image-related data (14) of the monitoring sensor arrangement (11) are being correlated with the calibration sensor data of the calibration sensor arrangement (23), in order to improve the accuracy of the calculation model (18) and/or to derive the absolute maximum particle filling level.

10. Method according to any one of the preceding claims, **characterized in that** in a calibration routine, a loading cycle (8) proceeds to and beyond the point of particle breakthrough, and that the particle filling level (17) calculated at the point of particle breakthrough is being stored as the absolute maximum particle filling level in the electronic process control (7).

11. Method according to any one of the preceding claims, **characterized in that** in an adjusting routine at least one parameter of the clarification setup (1) is being adjusted by the electronic process control (7) based on the particle filling levels (17) of the centrifuge chambers (5) according to a predefined adjusting strategy, and/or, that in an automation routine, the electronic process control (7) initiates centrifugation cycles according to a predefined automation strategy based on the calculated particle filling level (17).

12. Method according to claim 11, **characterized in that** a nominal maximum particle filling level is stored in the electronic process control (7) and that the nominal maximum particle filling level is preferably below the absolute maximum particle filling level by a predefined upper offset and that, when the nominal maximum particle filling level is approached during the loading cycle (8), according to the automation strategy, the loading cycle (8) is terminated and, preferably, the washing cycle (9) and/or the discharging cycle (10) is initiated by the electronic process control (7), and/or, that a nominal zero particle filling level is stored in the electronic process control (7) and that the nominal zero particle filling level is above the absolute zero particle filling level by a predefined lower offset and that, when the nominal zero particle filling level is approached during the discharging cycle (10), according to the automation strategy, the discharging cycle (10) is terminated and, preferably, the loading cycle (8) is initiated by the electronic process control (7).

13. Method according to any one of the preceding claims, **characterized in that** the bioprocess installation (2) comprises a downstream setup (34) with at least one downstream unit (35), and that in an adapting routine, the electronic process control (7) adapts at least one parameter of the downstream setup (34) according to the image-related data (14) based on a predefined adapting strategy.

14. Method according to any one of the preceding claims, **characterized in that** in an analyzing routine, an irregularity state attributed to the presence of impurities, preferably contaminating organisms, is detected based on predefined irregularity features in the optical image (15).

15. Clarification setup (1) of a bioprocess installation (2) for performing the method according to any one of the preceding claims, wherein the clarification setup (1) comprises a fluidized bed centrifuge (3) for the clarification of a cell broth by centrifugation and a pumping arrangement (4) assigned to the fluidized bed centrifuge (3), wherein the fluidized bed centrifuge (3) comprises at least one centrifuge chamber (5), which is being turned around a geometrical centrifuge axis (6), wherein the bioprocess installation (2) comprises an electronic process control (7) for controlling at least the fluidized bed centrifuge (3) and the pumping arrangement (4), wherein the fluidized bed centrifuge (3) is being operated in a forward operation for a particle loading cycle (8) and/or a particle washing cycle (9) and in a backward operation for a particle discharging cycle (10), wherein in the case where a particle loading cycle (8) is provided, during the loading cycle (8), cell broth loaded into the centrifuge chamber (5) proceeds to form a growing particle accumulation in the centrifuge chamber (5), wherein the clarification setup (1) comprises a monitoring sensor arrangement (11) with at least one optical sensor (12) for producing monitoring sensor data, which are being transmitted to the electronic process control (7),
**characterized in**
**that** in a monitoring routine (13), image-related data (14) representing an optical image (15) of the centrifuge chamber content (16) are being produced by the monitoring sensor arrangement (11) and a particle filling level (17) is being calculated by the electronic process control (7) from the image-related data (14) based on a calculation model (18).

16. Use of a centrifuge chamber (5), which as such is made of a translucent material, for performing the method according to any one of the claims 1 to 14.
